# FASCICULE DE BREVET EUROPEEN

(11) **EP 0 781 345 B1**
(45) Date de publication et mention de la délivrance du brevet: **16.12.1998**
(21) Numéro de dépôt: 95930580.6
(22) Date de dépôt: 13.09.1995
(51) Int. Cl.: C12P 7/42, C12P 7/24

(54) **PROCEDE D'OBTENTION D'ACIDE VANILLIQUE ET DE VANILLINE PAR BIOCONVERSION PAR UNE ASSOCIATION DE MICROORGANISMES FILAMENTEUX**
VERFAHREN ZUR GEWINNUNG VON VANILLINSÄURE UND VANILLIN DURCH BIOKONVERSION MIT EINER KOMBINATION VON FILAMENTÖSEN MIKROORGANISMEN
METHOD FOR OBTAINING VANILLIC ACID AND VANILLIN BY BIOCONVERSION BY AN ASSOCIATION OF FILAMENTOUS MICROORGANISMS

(30) Priorité: 13.09.1994 FR 9410889
(43) Date de publication de la demande: 02.07.1997
(73) Titulaire: INSTITUT NATIONAL DE LA RECHERCHE AGRONOMIQUE (I.N.R.A.), 75341 Paris Cédéx 07 (FR)
(72) Inventeur: LESAGE-MEESSEN, Laurence, F-13008 Marseille (FR); DELATTRE, Michel Bâtiment F, F-13009 Marseille (FR); HAON, Mireille, F-13016 Marseille (FR); ASTHER, Marcel, F-13006 La Ciotat (FR)
(74) Mandataire: Vialle-Presles, Marie José
(86) Numéro de dépôt international: FR9501173
(87) Numéro de publication internationale: WO9608576

(56) Documents cités:
- EP-A- 0 453 368
- US-A- 5 128 253
- CHEMICAL ABSTRACTS, vol. 120, no. 19, 9 Mai 1994 Columbus, Ohio, US; abstract no. 239831, MIDDELHOVEN, WOUTER J. 'Catabolism of benzene compounds by ascomycetous and basidiomycetous yeasts and yeastlike fungi: a literature review and an experimental approach' & ANTONIE VAN LEEUWENHOEK (1993), 63(2), 125-44 CODEN: ALJMAO;ISSN: 0003-6072,
- CHEMICAL ABSTRACTS, vol. 121, no. 23, 5 Décembre 1994 Columbus, Ohio, US; abstract no. 276417, FALCONNIER, B. ET AL 'Vanillin as a product of ferulic acid biotransformation by the white-rot fungus Pycnoporus cinnabarinus I-937: Identification of metabolic pathways' & J. BIOTECHNOL. (1994), 37(2), 123-32 CODEN: JBITD4;ISSN: 0168-1656,

## Description

La présente Invention est relative à l'obtention d'acide vanillique et de vanilline par bioconversion.

Actuellement, la vanilline est l'arôme le plus utilisé dans les industries agro-alimentaires. Or, la production de vanilline naturelle à partir de gousses de vanille ne couvre que 20% des besoins du marché et son prix de revient est de l'ordre de 25 000F/kg.

La vanilline peut également être obtenue par synthèse chimique ; toutefois, cette méthode d'obtention, si elle convient pour la fabrication des parfums et des cosmétiques, peut soulever dans les industries agro-alimentaires des difficultés d'ordre législatif. En outre les arômes de synthèse ont tendance par ailleurs à être moins appréciés par les consommateurs que les arômes d'origine naturelle.

C'est pourquoi l'on cherche à obtenir des composés aromatiques produits grâce à des procédés biologiques, qui mettent en oeuvre des microorganismes (bactéries, levures, champignons), des cellules animales ou végétales ou leurs systèmes enzymatiques.

La vanilline est produite par certains végétaux et micooorganismes, en particulier des champignons, où elle constitue un des produits de dégradation de précurseurs à noyau aromatique (acide férulique et acide vanillique).

La Demande de brevet européen 453 368 au nom de la Société PERNOD-RICARD, décrit la production de vanilline naturelle par bioconversion d'acide férulique ou d'acide vanillique, en présence d'un champignon filamenteux du groupe des Basidiomycètes, *Pycnoporus cinnabarinus.*

US-A-5128253 décrit la production de vanilline à partir de l'acide férulique en utilisant une souche d'un microorganisme, tel que Aspergillus niger et mentionne l'inclusion du cellobiose.

Chez ce champignon, quatre voies métaboliques de transformation de l'acide férulique ont été identifiées :
- **Voie 1** : l'acide férulique est réduit en aldéhyde coniférylique puis en alcool coniférylique ; différents dimères sont ensuite formés à partir de ce composé. Cette voie est mineure.
- **Voie 2** : il y a coupure de la chaîne propénoïque de l'acide férulique avec perte de deux carbones et formation d'acide vanillique.
- **Voie 3** : l'acide vanillique résultant de la voie 2 est réduit en vanilline par la Réductase I. La vanilline produite peut ensuite être réduite par la Réductase II en alcool vanillique.
- **Voie 4** : l'acide vanillique résultant de la voie 2 est hydroxylé et décarboxylé en méthoxyhydroquinone, par l'action d'une vanillate hydroxylase intracellulaire.

Dans les conditions décrites dans la Demande EP 453 368, la production de vanilline par P. *cinnabarinus* MIC11, à partir de 300 mg/l d'acide férulique est au maximum de l'ordre de 45 mg/l (rendement molaire de conversion de 20,5 %), et à partir de 300 mg/l d'acide vanillique, cette production est au maximum de l'ordre de 81,4 mg/l (rendement molaire de conversion de 31%).

La présente Invention a pour but d'améliorer le rendement de la production de vanilline naturelle par bioconversion à partir de précurseurs à noyau aromatique (acide férulique et acide vanillique). Dans ce but, les Inventeurs ont cherché à améliorer le rendement de conversion de l'acide férulique en acide vanillique, et le rendement de conversion de l'acide vanillique en vanilline.

La présente Invention a pour objet un procédé d'obtention de l'acide vanillique par bioconversion à partir de l'acide férulique, lequel procédé est caractérisé en ce que ladite bioconversion est effectuée en utilisant une culture comprenant au moins une souche d'un champignon filamenteux choisi dans le groupe constitué par les Ascomycètes et les Basidiomycètes, ou au moins une souche d'Actinomycète.

La présente Invention a également pour objet un procédé d'obtention de vanilline par bioconversion à partir de l'acide férulique, lequel procédé est caractérisé en ce qu'il comprend :
- une étape de transformation de l'acide férulique en acide vanillique par le procédé défini ci-dessus :
- une étape de transformation de l'acide vanillique en vanilline par au moins un champignon filamenteux de la classe des Basidiomycètes.

Selon une première variante du procédé d'obtention de vanilline conforme à l'Invention, les deux étapes sont effectuées séquentiellement. Dans ce cas le procédé conforme à l'Invention comprend:
- une étape au cours de laquelle on ajoute de l'acide férulique à une culture comprenant au moins une souche d'un champignon filamenteux choisi dans le groupe constitué par les Ascomycètes et les Basidiomycètes, ou au moins une souche d'Actinomycète, et l'on recueille l'acide vanillique produit ;
- une étape au cours de laquelle on ajoute l'acide vanillique produit au cours de la première étape à une culture comprenant au moins une souche d'un champignon filamenteux de la classe des Basidiomycètes, et l'on recueille la vanilline produite.

Pour mettre en oeuvre cette variante, les champignons utilisés dans chacune des étapes sont cultivés séparément jusqu'à l'obtention d'une biomasse possédant les capacités nécessaires à la bioconversion (c'est à dire une biomasse d'au moins 0,5 g de matière sèche par litre de culture), et les cultures obtenues sont ensuite utilisées successivement.

Selon une deuxième variante du procédé conforme à l'Invention, les deux étapes sont effectuées simultanément. Dans ce cas l'on ajoute de l'acide férulique à une culture comprenant, pour la transformation de l'acide férulique en acide vanillique, au moins une souche d'un champignon filamenteux choisi dans le groupe constitué par les Ascomycètes et les Basidiomycètes, ou au moins une souche d'Actinomycète, et, pour la transformation de l'acide vanillique en vanilline, au moins une souche de Basidiomycète qui peut être identique à ou différente de celle utilisée pour transformer l'acide férulique en acide vanillique, et l'on recueille la vanilline produite.

Pour mettre en oeuvre cette deuxième variante, les champignons utilisés pour la transformation de l'acide férulique en acide vanillique et ceux utilisés pour la transformation de l'acide vanillique en vanilline sont cultivés séparément jusqu'à l'obtention d'une biomasse possédant les capacités nécessaires à la bioconversion, et les cultures obtenues sont ensuite regroupées pour obtenir une co-culture permettant de réaliser la bioconversion en une seule étape.

Pour la bioconversion de l'acide férulique en acide vanillique, des Ascomycètes préférés appartiennent aux genres *Eurotium, Penicillium,* et *Aspergillus ;* des Basidiomycètes préférés appartiennent aux genres *Bjerkandera, Nidula, Nidularia, Phanerochaete, Pycnoporus, Trametes, Lentinus,* et *Ischnoderma ;* des Actinomycètes préférés appartiennent aux *Streptomyces.*

Pour la bioconversion de l'acide vanillique en vanilline, des Basidiomycètes préférés appartiennent aux genres *Bjerkandera, Nidula, Nidularia, Phanerochaete, Pycnoporus, Trametes, Lentinus,* et *Ischnoderma.*

Le Tableau I ci-après mentionne, de façon non limitative, quelques espèces convenant à la mise en oeuvre du procédé selon l'Invention, et quelques souches testées par les Inventeurs, et qui se sont avérées particulièrement performantes dans ce cadre.

**TABLEAU I**

| Espèces | Exemple de souches utilisées |
|---|---|
| Actinomycètes : | |
| *- Streptomyces setonii* Ascomycètes : | ATCC 25497 |
| *- Eurotium chevalieri* | |
| *- Penicillium verrucosum cyclopium* | |
| *- Aspergillus oryzae* | |
| *- Aspergillus versicolor* | |
| *- Aspergillus niger* Basidiomycètes | LMTC N° 2.7 (CNCM I-1472) |
| *- Ischnoderma benzoïmum* | CBS 250.30 |
| *- Bjerkandera adusta* | CBS 595.79 |
| *- Nidula niveo-tomentosa* | ATCC 38357 |
| *- Phanerochaete sordida* | IHEM 3730 |
| *- Phanerochaete chrysosporium* | MIC 247 (CNCM I-1471) |
| *- Pycnoporus cinnabarinus* | MUCL 38467 |
| *- Trametes pini* | CBS 210.36 |
| *- Lentinus edodes* | CBS 454.59 |

Il est possible, selon les cas, de se procurer les différentes souches citées, auprès de la collection MUCL, 3 place Croix du Sud, 1348, Louvain La Neuve, Belgique, auprès de la collection IHEM, 14 rue J. Wytsman, 1050, Bruxelles, Belgique, auprès de la collection CBS, Oosterstraat 1, Postbus 273, NL-3740 AG Baarn, et auprès de la collection ATCC, 12301 Parklawn Drive, Rockville, Maryland 20852 USA.

D'autre part, la souche *d'Aspergillus niger* LMTC 2.7 et la souche de *Phanerochaete chrysosporium* MIC 247 ont été déposées le 31 août 1994 auprès de la CNCM (Collection Nationale de Cultures de Microorganismes) tenue par l'Institut Pasteur, 26 rue du Docteur Roux, à Paris, sous les numéros respectifs I-1472 et I-1471.

Les organismes peuvent être cultivés sous forme de cellules libres, ou sous forme de cellules immobilisées sur un support hydrophile ou hydrophobe, de préférence rugueux.

Les cultures sont effectuées, de manière classique, à partir d'un inoculum qui peut être constitué par des spores, par des fragments de mycélium, ou par une préculture de mycélium. Le milieu de culture comporte une source de carbone, au moins une source d'azote, des sels minéraux, et est additionné d'extrait de levure. Les constituants de ce milieu peuvent être ceux qui sont classiquement utilisés pour la culture de l'espèce de champignon concernée.

Toutefois, les Inventeurs ont constaté que lorsque la source de carbone utilisée comprend ou est constituée par au moins un phospholipide, on observe une accélération de la bioconversion, en particulier en ce qui concerne la bioconversion de l'acide férulique en acide vanillique.

Avantageusement, le milieu de culture comprend au moins un phospholipide choisi dans le groupe constitué par la phosphatidylcholine, la lysophosphatidylcholine, la phosphatidyléthanolamine, 1'acylphosphatidyléthanolamine, le phosphatidylinositol, et l'acide phosphatidique.

Préférentiellement ce phospholipide ou mélange de phospholipides est utilisé à une concentration comprise entre 0,1 et 20 g/l.

Par exemple on peut utiliser un mélange de phospholipides de soja comprenant :
- 12 % de phosphatidylcholine et lysophosphatidylcholine ;
- 31 % de phosphatidyléthanolamine et acylphosphatidyléthanolamine ;
- 27 % de phosphatidylinositol ;
- 30 % d'acide phosphatidique.

Pour améliorer le rendement de bioconversion de l'acide férulique en acide vanillique, on peut également activer la bêta-oxydation peroxysomale.

Les Inventeurs ont en outre observé que l'utilisation de cellobiose comme seule source de carbone du champignon, ou en complément d'une autre source de carbone, par exemple d'un sucre tel que le maltose, permet de limiter très significativement la production de méthoxyhydroquinone, et donc d'augmenter le rendement de bioconversion de l'acide vanillique en vanilline.

Selon un mode de mise en oeuvre préféré du procédé conforme à l'Invention, la source de carbone utilisée comprend ou est constituée par du cellobiose.

Selon une disposition préférée de ce mode de mise en oeuvre, le milieu de culture comprend du cellobiose.

Le cellobiose peut être ajouté dès le début de la culture, à une concentration comprise entre 0,5 g/l et 10 g/l, préférentiellement 5 g/l, et/ou peu de temps avant, ou simultanément à l'addition de l'acide férulique ou de l'acide vanillique, à une concentration comprise dans ce cas entre 0,5 g/l et 5 g/l, préférentiellement 2,5 g/l.

Pour initier la bioconversion, on additionne à la culture le précurseur, à savoir soit l'acide férulique, puis l'acide vanillique, dans le cas de la première variante du procédé, soit l'acide férulique, dans le cas de la deuxième variante du procédé.

Il est avantageux d'ajouter en même temps que le précurseur, une quantité, comprise entre 0,01 et 5 g/l d'au moins un produit constituant une source de carbone, ou une source d'azote, ou de sels minéraux, ou de lipides, ou d'un mélange de ces différents produits.

On citera parmi les sources de carbone utilisables dans ce but, le maltose, le cellobiose, l'acide galacturonique, le xylose, le rhamnose, l'arabinose ; parmi les sources d'azote, le tartrate diammonium, l'extrait de levure ; parmi les sels minéraux, les sels de calcium, de potassium, de magnésium ; parmi les lipides, les acides gras émulsifiés, et les phospholipides.

L'acide férulique est une matière première aisément disponible, et peu coûteuse. Il est présent par exemple dans la fraction pariétale de sous-produits agricoles tels que les pulpes de betterave (0,9% du poids sec) ou les sons de céréales (2,0% du poids sec dans les sons de maïs). L'acide vanillique peut en particulier être produit à partir de l'acide férulique au cours de la première étape du procédé conforme à l'Invention.

L'acide férulique peut être utilisé sous forme libre ou bien sous forme liée ; on entend par acide férulique lié un ester d'un sucre ou d'un oligosaccharide avec l'acide férulique.

L'acide férulique libre ou l'acide vanillique peuvent être ajoutés tels quels, sous forme de cristaux, ou sous forme d'une solution de leurs sels de sodium, potassium, ou ammonium.

Selon un mode de mise en oeuvre préféré du procédé conforme à l'invention, l'acide férulique ou l'acide vanillique sont ajoutés à raison de 0,1 à 2 g/l de culture, de préférence 0,3 g/l. Si l'on utilise l'acide férulique lié, ou bien un sel d'acide férulique, ou d'acide vanillique, on ajustera la concentration de manière à ce qu'elle corresponde à la gamme de concentrations en acide férulique ou en acide vanillique indiquée ci-dessus.

Selon un autre mode de mise en oeuvre préféré du procédé conforme à l'invention, l'ajout de l'acide férulique ou de l'acide vanillique s'effectue après 12 à 96 h de culture. Toutefois, lorsque l'ajout en une seule fois aboutirait à des concentrations toxiques pour le champignon, à savoir des concentrations supérieures à 2 g/l pour l'acide férulique et à 2 g/l pour l'acide vanillique, des ajouts séquentiels ou en continu seront effectués.

Les Inventeurs ont en outre observé qu'il était possible et particulièrement avantageux, de mettre en oeuvre le procédé selon l'Invention en présence d'une résine non-ionique, de préférence de type hydrophobe. En effet, non seulement l'acide vanillique, mais également la vanilline sont toxiques pour les champignons, à des concentrations supérieures à 2 g/l. L'utilisation de résine permet de piéger ces métabolites et de maintenir leurs concentrations en dessous du seuil de toxicité.

D'autre part, l'utilisation de résine pour piéger la vanilline permet également d'éliminer celle-ci du mélange réactionnel avant qu'elle soit réduite en alcool vanillique.

L'acide vanillique ou la vanilline fixés sur la résine peuvent être récupérés, par élution avec un solvant approprié, tel par exemple que l'éthanol.

Des résines non-ioniques pouvant être utilisées sont, à titre d'exemple non-limitatif, les suivantes :
- Résines AMBERLITE^{®} : XAD2 ; XAD4 ; XAD7 ;
- Résine DUOLITE^{®} : XAD761 ;
- Résine DOWEX^{®} : S112

Les résines XAD761 et S112 sont préférées pour fixer l'acide vanillique, et les résines XAD2, XAD4, XAD7, et S112, sont préférées pour fixer la vanilline.

A titre d'exemple, la résine XAD2 permet de fixer 98% de la vanilline ; la capacité d'adsorption de cette résine est de l'ordre de 20 mg de vanilline par gramme de résine.

Préférentiellement, l'ajout de résine est effectué dès l'apparition de la vanilline.

L'ajout de résine peut s'effectuer, par exemple, soit directement dans le récipient de culture, soit par circulation du milieu de culture dans une boucle externe contenant la résine.

Pour éliminer l'acide vanillique et la vanilline du milieu de culture et les récupérer au fur et à mesure de leur production, il est également possible de faire appel à d'autres techniques, telles que l'ultrafiltration, l'osmose inverse ou la capture sur charbon actif.

Pour améliorer de façon significative le rendement de bioconversion de l'acide vanillique en vanilline, on peut également choisir une souche de basidiomycète qui ne forme que très peu d'alcool vanillique, et accumule la vanilline produite.

Il s'agit par exemple de basidiomycètes de l'ordre des Nidulariales.

La vanilline produite devenant très rapidement toxique pour le métabolisme du champignon, il est avantageux dans ce cas de la piéger en utilisant une résine, comme indiqué ci-dessus.

La présente Invention sera mieux comprise à l'aide du complément de description qui va suivre, qui se réfère à des exemples de mise en oeuvre du procédé conforme à l'Invention.

Il doit être bien entendu toutefois que ces exemples sont donnés uniquement à titre d'illustration de l'objet de l'Invention dont ils ne constituent en aucune manière une limitation.

### EXEMPLE 1 : Réalisation des cultures

### A) Champignons :

La composition du milieu de base est donnée ci-dessous :

| Composition du milieu : | |
|---|---|
| Maltose | 20 g/l |
| Tartrate diammonium | 1,842 g/l |
| KH₂PO₄ | 0,2 g/l |
| CaCl₂, 2 H₂O | 0,0132 g/l |
| MgSO₄, 7H₂O | 0,5 g/l |
| Extrait de levure | 0,5 g/l |

Le milieu est stérilisé par autoclavage 20 min à 120 C.

L'inoculation est effectuée par des fragments mycéliens, des spores (2.10⁵ spores/ml), ou une préculture de mycélium, comme décrit dans la Demande EP 453 368.

Après inoculation, les cultures sont incubées à 30°C et soumises à une agitation de 120 tours/min.

Après 12 à 96 h de culture, l'acide férulique ou l'acide vanillique (dans le cas de la mise en oeuvre du procédé en deux étapes) sont ajoutés. Ils sont utilisés sous forme de sel en solution dans l'eau ; la solution a été préalablement stérilisée par filtration (membrane de 0,2µm). La solution est ajoutée en quantité suffisante pour obtenir une concentration finale correspondant à 0,3 g d'acide férulique ou d'acide vanillique par litre de culture.

### B) Actinomycètes :

La composition du milieu de base est la suivante :

| | |
|---|---|
| Glycérol | 5 g/l |
| Extrait de levure | 2 g/l |

Le milieu est stérilisé par autoclavage 20 min à 120°C.

L'inoculation est effectuée par des fragments de mycélium aérien prélevés sur un milieu solide malt/agar.

Après inoculation, les cultures sont incubées à 30°C et soumises à une agitation de 120 tours/min.

L'acide férulique, sous forme de sel, est ajouté en tout début de culture, à la concentration de 1 g/l.

### C) Suivi de la bioconversion :

La bioconversion, au cours de la mise en oeuvre du procédé conforme à l'Invention est suivie par le dosage des métabolites produits. Pour procéder à ce dosage, un aliquot du milieu de culture est prélevé stérilement à intervalles de temps réguliers. Cet aliquot est ensuite filtré (membrane de 0.2 µn) et une analyse par HPLC du filtrat est réalisée, pour détecter et doser les métabolites produits.

### Conditions d'analyse HPLC

- phase inverse = colonne BONDAPACK C18
- solvant : CH₃COOH 0,01 % dans H₂O/méthanol
- détection en UV à 280 nm

### EXEMPLE 2 : Bioconversion de l'acide férylique en acide vanillique

### A) Par un champignon

La souche *d'Aspergillus niger* MIC 373, déposée le 31 août 1994 auprès de la Collection Nationale de Culture de Microorganismes sous le numéro I-1472, a été mise en culture comme indiqué à l'exemple 1 ci-dessus.

L'acide férulique a été ajouté en continu à raison de 430 mg/l par 24 heures.

L'acide férulique et ses métabolites sont dosés comme décrit à l'exemple 1 ci-dessus.

Les résultats obtenus après 15 jours de croissance de la culture sont indiqués dans le Tableau II ci dessous.

**TABLEAU II**

| | | | |
|---|---|---|---|
| 1 | 2 | 3 | 4 |
| 5055 | 3600 | 109 | 82 |
| Légende du Tableau II : Colonne 1 : Acide férulique consommé (mg/l); Colonne 2 : Acide vanillique produit (mg/l); Colonne 3 : Methoxyhydroquinone produite (mg/l) ; Colonne 4 : Rendement molaire (%) ; le rendement molaire est défini comme le nombre de moles d'acide vanillique produites pour 100 moles d'acide férulique consommées. | | | |

On observe une consommation totale de l'acide férulique ajouté. Celui-ci est transformé en grande majorité (82%) en acide vanillique qui est faiblement (2%) métabolisé en méthoxyhydroquinone, et pas du tout en vanilline et alcool vanillique.

### B) Par un actinomycète :

La souche de *Streptomyces setonii* ATCC 25497 a été mise en culture comme indiqué à l'exemple 1 ci-dessus.

L'acide férulique, sous forme de sel, est ajouté en tout début de culture, à la concentration de 1 g/l.

L'acide férulique et ses métabolites sont dosés comme décrit à l'exemple 1 ci-dessus.

Les résultats obtenus après 100 heures de croissance de la culture sont indiqués dans le Tableau III ci dessous.

**TABLEAU III**

| | | |
|---|---|---|
| 1 | 2 | 3 |
| 880 | 332 | 43 |
| Légende du Tableau III : Colonne 1 : Acide férulique consommé (mg/l) ; Colonne 2 : Acide vanillique produit (mg/l) ; Colonne 3 : Rendement molaire (%). | | |

### EXEMPLE 3 : Activation de la bioconversion de l'acide férulique en acide vanilique par ajout de phospholipides de soja

La source de phospholipides utilisée est le NAT 89 fourni par la Société Natterman Phospholipid Gmbh (Cologne, Allemagne). La composition du NAT 89 est la suivante :
12 % de phosphatidylcholine et de lysophosphatidylcholine
31 % de phosphatidylethanolamine et acyl-phosphatidylethanolamine
27 % de phosphatidylinositol
30 % d'acide phosphatidique

Le NAT89 (20 g/l) a été utilisé comme source de carbone à la place du maltose dans les cultures *d'Aspergillus niger.* Les résultats obtenus montrent une bioconversion plus rapide de l'acide férulique en acide vanillique.

### EXEMPLE 4 : Activation de la bioconversion de l'acide vanillique en vanilline par l'utilisation de résine

Pour illustrer cet exemple, une souche représentative d'une espèce de Basidiomycète produisant principalement de l'alcool vanillique a été choisie.

Il s'agit de la souche MIC 247 de *Phanerochaete chrysosporium,* déposée le 31 août 1994 auprès de la Collection Nationale de Culture de Microorganismes sous le numéro I-1471. Cette souche a été mise en culture comme indiqué à l'exemple 1 ci-dessus.

L'acide vanillique a été ajouté séquentiellement, à savoir : 0,3 g/l au bout de 3 jours de culture, puis 0,3 g/l tous les jours.

Pour l'expérience 2, de la résine XAD2 (AMBERLITE) stérile est ajoutée à raison de 10% (poids/volume) après 3 jours + 6h de culture du champignon, soit 6 h après l'ajout d'acide vanillique (à 300 mg/l).

L'acide vanillique et ses métabolites sont dosés comme décrit à l'exemple 1 ci-dessus.

Les résultats, indiqués dans le Tableau IV ci-dessous, sont donnés après 4 jours et 7 jours (Expérience 1), ou après 6 jours (expérience 2), de croissance de la culture.

**TABLEAU IV**

| 1 | 2 | 3 | 4 | 5 | 6 |
|---|---|---|---|---|---|
| N°1 | | | | | |
| 4 j | 277 | 125 | 89 | 17 | 50 |
| | | | | | |
| 7 j | 1545 | 3 | 762 | 175 | 0,2 |
| | | | | | |

| N°2 | | | | | |
|---|---|---|---|---|---|
| 6j | 838 | 628 | 51 | 86 | 82 |
| Légende du Tableau IV : Colonne 1 : Numéro de l'expérience, et âge de la culture ; Colonne 2 : Acide vanillique consommé (mg/l) ; Colonne 3 : Vanilline produite (mg/l) ; Colonne 4 : Alcool vanillique produit (mg/l) ; Colonne 5 : Méthoxyhydroquinone produite (mg/l) ; Colonne 6 : Rendement molaire (%) ; le rendement molaire est ici défini comme le nombre de moles de vanilline produites pour 100 moles d'acide vanillique consommées. | | | | | |

Dans l'expérience 1, on observe dès le quatrième jour une forte production d'alcool vanillique associée à la production de vanilline. Au septième jour, toute la vanilline produite a été métabolisée en alcool vanillique.

Dans l'expérience 2, la vanilline a été fixée par la résine au fur et à mesure de sa production (94 % de la vanilline produite a été fixée sur la résine utilisée), avant qu'elle ne soit transformée en alcool vanillique.

### EXEMPLE 5 : Activation de la bioconversion de l'acide vanillique en vanilline par l'utilisation de cellobiose

Pour illustrer cet exemple, une souche représentative d'une espèce de Basidiomycète produisant principalement de la méthoxyhydroquinone a été choisie.

Il s'agit de la souche MUCL 38467 de *Pycnoporus cinnabarinus.* Cette souche a été mise en culture comme indiqué à l'exemple 1 ci-dessus.

L'acide vanillique a été ajouté séquentiellement, à savoir : 0,3 g/l au bout de 3 jours de culture, puis 0,3 g/l tous les jours.

Pour l'expérience 1, la source de carbone utilisée est le maltose, à une concentration de 20 g/l.

Pour l'expérience 2, la source de carbone utilisée est le cellobiose, à raison de 5 g/l.

L'acide vanillique et ses métabolites sont dosés, comme décrit à l'exemple 1 ci-dessus.

Les résultats, après 7 jours de croissance de la culture, sont indiqués dans le Tableau V ci-dessous.

**TABLEAU V**

| 1 | 2 | 3 | 4 | 5 | 6 |
|---|---|---|---|---|---|
| N°1 | 1198 | 166 | 709 | 30 | 15 |
| N°2 | 941 | 481 | 62 | 70 | 59 |
| Légende du Tableau V : Colonne 1 : Numéro de l'expérience ; Colonne 2 : Acide vanillique consommé (mg/l) ; Colonne 3 : Vanilline produite (mg/l) ; Colonne 4 : Methoxyhydroquinone produite (mg/l) ; Colonne 5 : Alcool vanillique produit (mg/l) ; Colonne 6 : Rendement molaire (%) ; le rendement molaire est défini comme le nombre de moles de vanilline produites pour 100 moles d'acide vanillique consommées. | | | | | |

Dans l'expérience 1, on observe après 7 jours de culture des productions élevées en vanilline mais aussi en méthoxyhydroquinone.

Dans l'expérience 2, l'utilisation de cellobiose comme source de carbone, a permis de limiter très significativement la production de méthoxyhydroquinone, et d'augmenter celle de vanilline.

Dans une deuxième série d'expériences décrites ci-dessous, où le maltose est utilisé comme source de carbone principale, l'ajout de cellobiose avant l'ajout du précurseur permet également de limiter très significativement la production de méthoxyhydroquinone et d'augmenter celle de vanilline.

Les milieux et conditions de culture suivants ont été utilisés:
- Maltose 1 : Seule source de carbone : maltose à 20 g/l.
- Maltose 2 : Source de carbone : maltose à 20 g/l + addition de 0,25% (p/v) de cellobiose après 3 jours d'incubation, 2 h avant l'ajout d'acide vanillique.
- Maltose 3 : Source de carbone : maltose à 20 g/l + addition de 0,35% (p/v) de cellobiose après 3 jours d'incubation, 2 h avant l'ajout d'acide vanillique.
- Maltose 4 : Source de carbone : maltose à 20 g/l + addition de 0,5% (p/v) de cellobiose après 3 jours d'incubation, 2 h avant l'ajout d'acide vanillique.
- Maltose 2C : Source de carbone : maltose à 20 g/l + addition de 0,25% (p/v) de cellobiose après 3 jours, 4 jours, 5 jours, et 6 jours d'incubation, chaque ajout précédant de 2 heures l'ajout d'acide vanillique.
- Maltose 3C : Source de carbone : maltose à 20 g/l + addition de 0,35% (p/v) de cellobiose après 3 jours, 4 jours, 5 jours, et 6 jours d'incubation, chaque ajout précédant de 2 heures l'ajout d'acide vanillique.
- Maltose 4C : Source de carbone : maltose à 20 g/l + addition de 0,5% (p/v) de cellobiose après 3 jours, 4 jours, 5 jours, et 6 jours d'incubation, chaque ajout précédant de 2 heures l'ajout d'acide vanillique.

Les résultats sont indiqués dans le Tableau VI ci-dessous.

**TABLEAU VI**

| 1 | 2 | 3 | 4 | 5 | 6 |
|---|---|---|---|---|---|
| Maltosel | 1198 | 166 | 709 | 30 | 14 |
| Maltose2 | 1199 | 342 | 466 | 26 | 28 |
| Maltose3 | 1192 | 447 | 350 | 27 | 37 |
| Maltose4 | 1195 | 388 | 393 | 25 | 32 |
| Maltose2C | 1198 | 583 | 541 | 76 | 49 |
| Maltose3C | 1198 | 457 | 526 | 72 | 51 |
| Maltose4C | 1197 | 642 | 476 | 62 | 54 |
| Légende du Tableau VI : Colonne 1 : Numéro de l'expérience ; Colonne 2 : Acide vanillique consommé (mg/l) ; Colonne 3 : Vanilline produite (mg/l) ; Colonne 4 : Methoxyhydroquinone produite (mg/l) ; Colonne 5 : Alcool vanillique produit (mg/l) ; Colonne 6 : Rendement molaire (%) ; le rendement molaire est ici défini comme le nombre de moles de vanilline produites pour 100 moles d'acide vanillique consommées. | | | | | |

### Exemple 6 : Utilisation d'une souche produisant principalement de la vanilline

Pour illustrer cet exemple, une souche représentative d'une espèce de Basidiomycète accumulant la vanilline a été choisie.
Il s'agit d'une souche de *Nidula niveo-tomentosa* qui est accessible auprès de l'ATCC sous le numéro 38357. Cette souche a été mise en culture comme indiqué à l'exemple 1 ci-dessus.

L'acide vanillique a été ajouté séquentiellement, à savoir : 0,3 g/l au bout de 3 jours de culture, puis 0,3 g/l tous les jours.

L'acide vanillique et ses métabolites sont dosés comme décrit à l'exemple 1 ci-dessus.

Les résultats, au 8ème jour de croissance de la culture, sont indiqués dans le Tableau VII ci-dessous.

**TABLEAU VII**

| 1 | 2 | 3 | 4 | 5 |
|---|---|---|---|---|
| 1252 | 444 | 20 | 334 | 39 |
| Légende du Tableau VII : Colonne 1 : Acide vanillique consommé (mg/l) ; Colonne 2 : Vanilline produite (mg/l) ; Colonne 3 : Alcool vanillique produit (mg/l) ; Colonne 4 : Méthoxyhydroquinone produite (mg/l) ; Colonne 5 : Rendement molaire (%). | | | | |

Cette souche accumulant la vanilline produite, très peu d'alcool vanillique est obtenu.

Pour optimiser l'utilisation de cette souche, on peut d'une part piéger la vanilline au fur et à mesure de sa production, par exemple en utilisant des résines comme décrit à l'exemple 4 ci-dessus, et d'autre part ajouter du cellobiose afin de limiter la quantité de méthoxyhydroquinone produite, comme décrit à l'exemple 5 ci-dessus.

## Revendications

1. Procédé d'obtention d'acide vanillique ou de vanilline, caractérisé en ce qu'il comprend au moins l'une des deux étapes suivantes :
- une étape de bioconversion d'acide férulique en acide vanillique par au moins un champignon filamenteux choisi dans le groupe constitué par les Ascomycètes et les Basidiomycètes, ou au moins une souche d'Actinomycète, le milieu de culture dudit champignon filamenteux ou dudit Actinomycète comprenant au moins un phospholipide ou un mélange de phospholipides ;
- une étape de bioconversion d'acide vanillique en vanilline, par au moins un champignon filamenteux de la classe des Basidiomycètes ladite étape étant effectuée dans un milieu comprenant du cellobiose et/ou une résine hydrophobe.

2. Procédé d'obtention de vanilline, comprenant la mise en oeuvre d'une étape de bioconversion d'acide férulique en acide vanillique par au moins un champignon filamenteux choisi dans le groupe constitué par les Ascomycètes et les Basidiomycètes, ou au moins une souche d'Actinomycète, et d'une étape de bioconversion d'acide vanillique en vanilline par au moins un champignon filamenteux de la classe des Basidiomycètes, caractérisé en ce qu'au moins l'une de ces étapes est mise en oeuvre dans les conditions définies dans la revendication 1.

3. Procédé d'obtention de vanilline selon la revendication 2, caractérisé en ce qu'il comprend :
- une étape au cours de laquelle on ajoute de l'acide férulique à une culture comprenant au moins une souche d'un champignon filamenteux choisi dans le groupe constitué par les Ascomycètes et les Basidiomycètes, ou au moins une souche d'Actinomycète, et l'on recueille l'acide vanillique produit ; et
- une étape au cours de laquelle on ajoute l'acide vanillique produit au cours de la première étape à une culture comprenant au moins une souche d'un champignon filamenteux de la classe des Basidiomycètes, et l'on recueille la vanilline produite.

4. Procédé d'obtention de vanilline selon la revendication 2, caractérisé en ce que l'on ajoute de l'acide férulique à une culture comprenant au moins une souche d'un champignon filamenteux choisi dans le groupe constitué par les Ascomycètes et les Basidiomycètes, ou au moins une souche d'Actinomycète, et l'on recueille la vanilline produite.

5. Procédé selon une quelconque des revendications 1 à 4, caractérisé en ce que l'on met en oeuvre au moins une souche choisie dans le groupe constitué par : les souches d'Ascomycètes appartenant aux genres *Eurotium, Penicillium,* et *Aspergillus ;* les souches de Basidiomycètes appartenant aux genres *Bjerkandera, Nidula, Nidularia, Phanerochaete, Pycnoporus, Trametes, Lentinus,* et *Ischnoderma,* et les souches d'Actinomycètes appartenant au genre *Streptomyces.*

6. Procédé selon la revendication 5, caractérisé en ce que l'on met en oeuvre une souche *d'Aspergillus niger* déposée le 31 août 1994 auprès de la CNCM, sous le numéro I-1472.

7. Procédé selon une quelconque des revendications 2 à 6, caractérisé en ce que l'on met en oeuvre, pour la deuxième étape au moins une souche choisie dans le groupe constitué par des Basidiomycètes appartenant aux genres *Bjerkandera, Nidula, Nidularia, Phanerochaete, Pycnoporus, Trametes, Lentinus,* et *Ischnoderma.*

8. Procédé selon la revendication 7, caractérisé en ce que l'on met en oeuvre une souche de *Phanerochaete chrysosporium* déposée le 31 août 1994 auprès de la CNCM, sous le numéro I-1471.

9. Procédé selon une quelconque des revendications 1 à 8, caractérisé en ce que le milieu de culture mis en oeuvre lors de l'étape de bioconversion d'acide férulique en acide vanillique comprend au moins un phospholipide choisi dans le groupe constitué par la phosphatidylcholine, la lysophosphatidylcholine, la phosphatidyléthanolamine, l'acylphosphatidyléthanolamine, le phosphatidylinositol, et l'acide phosphatidique.

10. Procédé selon une quelconque des revendications 1 à 9 caractérisé en ce que ledit phospholipide ou mélange de phospholipides est utilisé à une concentration comprise entre 0,1 et 20 g/l.

11. Procédé selon une quelconque des revendications 1 à 10, caractérisé en ce que le milieu mis en oeuvre lors de l'étape de bioconversion d'acide vanillique en vanilline comprend du cellobiose à une concentration comprise entre 0,5 g/l et 10 g/l.

12. Procédé selon la revendication 11, caractérisé en ce que ledit milieu comprend du cellobiose à une concentration d'environ 5g /l.

13. Procédé selon une quelconque des revendications 1 à 12, caractérisé en ce que l'acide férulique est ajouté à raison de 0,1 à 2 g/l.

14. Procédé selon une quelconque des revendications 1 à 13, caractérisé en ce que l'acide vanillique est ajouté à raison de 0,1 à 2 g/l.

15. Procédé selon une quelconque des revendications 1 à 14, caractérisé en ce que le milieu mis en oeuvre lors de l'étape de bioconversion d'acide vanillique en vanilline comprend une résine hydrophobe choisie dans le groupe constitué par les résines XAD2 Amberlite^{®}, XAD4 Duolite^{®}, XAD7 Dowex^{®}, XAD761 Duolite^{®}, et S112 Dowex^{®}.

16. Souche *d'Aspergillus niger* déposée le 31 août 1994 auprès de la CNCM, sous le numéro I-1472.

## Patentansprüche

1. Verfahren zum Gewinnen von Vanillinsäure oder Vanillin, dadurch **gekennzeichnet**, daß es wenigstens eine der folgenden Stufen umfaßt:
- eine Stufe der Biokonversion der Ferulasäure in die Vanillinsäure mittels wenigstens eines Fadenpilzes, ausgewählt aus der Gruppe bestehend aus Ascomyceten und Basidiomyceten oder wenigstens eines Actinomycetenstammes, wobei das Kulturmedium des Fadenpilzes oder des Actinomyceten wenigstens ein Phospholipid oder ein Phospholipidgemisch enthält;
- eine Stufe der Biokonversion der Vanillinsäure in Vanillin mittels wenigstens eines Fadenpilzes der Klasse der Basidiomyceten, wobei die Stufe in einem Medium durchgeführt wird, das Cellobiose und/oder ein hydrophobes Harz enthält.

2. Verfahren zum Gewinnen von Vanillin, umfassend das Durchführen einer Stufe der Biokonversion der Ferulasäure in Vanillinsäure mittels wenigstens eines Fadenpilzes, ausgewählt aus der Gruppe bestehend aus Ascomyceten und Basidiomyceten oder wenigstens eines Actinomycetenstammes, und eine Stufe der Biokonversion der Vanillinsäure in Vanillin mittels wenigstens eines Fadenpilzes der Klasse der Basidiomyceten, dadurch **gekennzeichnet**, daß wenigstens eine dieser Stufen unter Bedingungen, die in Anspruch 1 definiert wurden, durchgeführt wird.

3. Verfahren zum Gewinnen von Vanillin nach Anspruch 2, dadurch **gekennzeichnet**, daß es umfaßt:
- eine Stufe, in deren Verlauf man Ferulasäure zu einer Kultur gibt, die wenigstens einen Fadenpilzstamm, ausgewählt aus der Gruppe bestehend aus Ascomyceten und Basidiomyceten oder wenigstens einen Actinomycetenstamm, enthält und daß man das Vanillinsäureprodukt gewinnt; und
- eine Stufe, in deren Verlauf man das in der ersten Stufe erhaltene Vanillinsäureprodukt zu einer Kultur zugibt, die wenigstens einen Fadenpilzstamm der Klasse der Basidiomyceten enthält, und daß man das Vanillinprodukt gewinnt.

4. Verfahren zum Gewinnen von Vanillin nach Anspruch 2, dadurch **gekennzeichnet**, daß man Ferulasäure zu einer Kultur gibt, die wenigstens einen Fadenpilzstamm, ausgewählt aus der Gruppe bestehend aus Ascomyceten und Basidiomyceten, und wenigstens einen Actinomycetenstamm enthält, und daß man das Vanillinprodukt gewinnt.

5. Verfahren nach einem der Ansprüche 1 bis 4, dadurch **gekennzeichnet**, daß man wenigstens einen Stamm, ausgewählt aus der Gruppe bestehend aus: den Ascomycetenstämmen, die zu den Gattungen *Eurotium, Penicillium* und *Aspergillus* gehören, die Basidiomycetenstämme, die zu den Gattungen *Bjerkandera, Nidula, Nidularia, Phanerochaete, Pycnoporus, Trametes, Lentinus* und *Ischnoderma* gehören, und die Actinomycetenstämme, die zu der Gattung *Streptomyces* gehören, verwendet.

6. Verfahren nach Anspruch 5, dadurch **gekennzeichnet**, daß man einen *Asperqillus-niqer-Stamm* verwendet, der am 31. August 1994 mit der Nummer 1-1472 bei CNCM hinterlegt wurde.

7. Verfahren nach einem der Ansprüche 2 bis 6, dadurch **gekennzeichnet**, daß man für die zweite Stufe wenigstens einen Stamm, ausgewählt aus der Gruppe bestehend aus den Basidiomyceten verwendet, die zu den Gattungen *Bjerkandera, Nidula, Nidularia, Phanerochaete, Pycnoporus, Trametes, Lentinus* und *Ischnoderma* gehören.

8. Verfahren nach Anspruch 7, dadurch **gekennzeichnet**, daß man einen *Phanerochaete-chrysosporium-Stamm* verwendet, der am 31. August 1994 mit der Nummer I-1471 bei CNCM hinterlegt wurde.

9. Verfahren nach einem der Ansprüche 1 bis 8, dadurch **gekennzeichnet**, daß das Kulturmedium, das bei der Stufe der Biokonversion der Ferulasäure in die Vanillinsäure verwendet wird, wenigstens ein Phospholipid, ausgewählt aus der Gruppe bestehend aus Phosphatidylcholin, Lysophosphatidylcholin, Phosphatidylethanolamin, Acylphosphatidylethanolamin, Phosphatidylinosit und Phosphatitsäure, enthält.

10. Verfahren nach einem der Ansprüche 1 bis 9, dadurch **gekennzeichnet**, daß das Phospholipid oder das Phospholipidgemisch in einer Konzentration im Bereich von 0,1 bis 20 g/l verwendet wird.

11. Verfahren nach einem der Ansprüche 1 bis 10, dadurch **gekennzeichnet**, daß das Medium, das bei der Stufe der Biokonversion von Vanillinsäure in Vanillin verwendet wird, Cellobiose in einer Konzentration im Bereich von 0,5 g/l bis 10 g/l enthält.

12. Verfahren nach Anspruch 11, dadurch **gekennzeichnet**, daß das Medium Cellobiose in einer Konzentration von etwa 5 g/l enthält.

13. Verfahren nach einem der Ansprüche 1 bis 12, dadurch **gekennzeichnet**, daß die Ferulasäure zu 0,1 bis 2 g/l zugegeben wird.

14. Verfahren nach einem der Ansprüche 1 bis 13, dadurch **gekennzeichnet**, daß die Vanillinsäure zu 0,1 bis 2 g/l zugegeben wird.

15. Verfahren nach einem der Ansprüche 1 bis 14, dadurch **gekennzeichnet**, daß das Medium, das bei der Stufe der Biokonversion der Vanillinsäure in Vanillin verwendet wird, ein hydrophobes Harz, ausgewählt aus der Gruppe bestehend aus den Harzen XAD2 (Amberlite^{®}), XAD4 (Amberlite^{®}), XAD7 (Amberlite^{®}), XAD761 (Duolite^{®}) und S112 (Dowex^{®}) enthält.

16. *Aspergillus-niger-Stamm,* der am 31. August 1994 unter der Nummer 1-1472 bei CNCM hinterlegt wurde.

## Claims

1. Process for obtaining vanillic acid or vanillin, characterized in that it comprises at least one of the following two steps:
- a step of bioconversion of ferulic acid to vanillic acid by at least one filamentous fungus chosen from the group consisting of Ascomycetes and Basidiomycetes, or at least one actinomycetes strain, the culture medium of the said filamentous fungus or said actinomycete comprising at least one phospholipid or a mixture of phospholipids;
- a step of bioconversion of vanillic acid to vanillin by at least one filamentous fungus of the class Basidiomycetes, the said step being performed in a medium comprising cellobiose and/or a hydrophobic resin.

2. Process for obtaining vanillin, comprising the carrying out of a step of bioconversion of ferulic acid to vanillic acid by at least one filamentous fungus chosen from the group consisting of Ascomycetes and Basidiomycetes, or at least one actinomycetes strain, and a step of bioconversion of vanillic acid to vanillin by at least one filamentous fungus of the class Basidiomycetes, -characterized in that at least one of these steps is carried out under the conditions defined in Claim 1.

3. Process for obtaining vanillin according to Claim 2, characterized in that it comprises:
- a step during which ferulic acid is added to a culture comprising at least one strain of a filamentous fungus chosen from the group consisting of Ascomycetes and Basidiomycetes, or at least one actinomycete strain, and the vanillic acid produced; and
- a step during which the vanillic acid produced during the first step is added to a culture comprising at least one strain of a filamentous fungus of the class Basidiomycetes, and the vanillin produced is collected.

4. Process for obtaining vanillin according to Claim 2, characterized in that ferulic acid is added to a culture comprising at least one strain of a filamentous fungus chosen from the group consisting of Ascomycetes and Basidiomycetes, or at least one actinomycete strain, and the vanillin produced is collected.

5. Process according to any one of Claims 1 to 4, characterized in that at least one strain is employed chosen from the group consisting of: Ascomycetes strains belonging to the genera *Eurotium, Penicillium* and *Aspergillus;* Basidiomycetes strains belonging to the genera *Bjerkandera, Nidula, Nidularia, Phanerochaete, Pycnoporus, Trametes, Lentinus* and *Ischnoderma;* and actinomycetes strains belonging to the genus *Streptomyces.*

6. Process according to Claim 5, characterized in that an *Aspergillus niger* strain deposited on 31st August 1994 with the CNCM under the number 1-1472 is employed.

7. Process according to any one of Claims 2 to 6, characterized in that, for the second step, at least one strain is employed chosen from the group consisting of Basidiomycetes belonging to the genera *Bjerkandera, Nidula, Nidularia, Phanerochaete, Pycnoporus, Trametes, Lentinus* and *Ischnoderma.*

8. Process according to Claim 7, characterized in that a *Phanerochaete chrysosporium* strain deposited on 31st August 1994 with the CNCM under the number I-1471 is employed.

9. Process according to any one of Claims 1 to 8, characterized in that the culture medium employed during the step of bioconversion of ferulic acid to vanillic acid comprises at least one phospholipid chosen from the group consisting of phosphatidylcholine, lysophosphatidylcholine, phosphatidylethanolamine, acylphosphatidylethanolamine, phosphatidylinositol and phosphatidic acid.

10. Process according to any one of Claims 1 to 9, characterized in that the said phospholipid or mixture of phospholipids is used at a concentration of between 0.1 and 20 g/l.

11. Process according to any one of Claims 1 to 10, characterized in that the medium employed during the step of bioconversion of vanillic acid to vanillin comprises cellobiose at a concentration of between 0.5 g/l and 10 g/l.

12. Process according to Claim 11, characterized in that the said medium comprises cellobiose at a concentration of approximately 5 g/l.

13. Process according to any one of Claims 1 to 12, characterized in that ferulic acid is added in the proportion of 0.1 to 2 g/l.

14. Process according to any one of Claims 1 to 13, characterized in that vanillic acid is added in the proportion of 0.1 to 2 g/l.

15. Process according to any one of Claims 1 to 14, characterized in that the medium employed during the step of bioconversion of vanillic acid to vanillin comprises a hydrophobic resin chosen from the group consisting of the resins XAD2, XAD4, XAD7, XAD761 and S112.

16. *Aspergillus niger* strain deposited on 31st August 1994 with the CNCM under the number I-1472.
